# EUROPÄISCHE PATENTSCHRIFT

(11) **EP 1 146 042 B1**
(45) Veröffentlichungstag und Bekanntmachung des Hinweises auf die Patenterteilung: **06.08.2003**
(21) Anmeldenummer: 01106212.2
(22) Anmeldetag: 14.03.2001
(51) Int. Cl.: C07D 223/22

(54) **Reinigungsverfahren zur Herstellung von hochreinem 5H-Dibenzo-(b,f)-azepin**
Process for the purification of 5H-dibenzo-(b,f)-azepine
Procédé de purification de la 5h-dibenzo-(b,f)-azépine

(30) Priorität: 10.04.2000 AT 6002000
(43) Veröffentlichungstag der Anmeldung: 17.10.2001
(73) Patentinhaber: DSM Fine Chemicals Austria Nfg GmbH & Co KG, 4021 Linz (AT)
(72) Erfinder: Schitter, Regina, 4020 Linz (AT); Raml, Walter, 4202 Hellmonsödt (AT); Heu, Ferdinand, 4030 Linz (AT); Steinwender, Erich, 4040 Linz (AT)
(74) Vertreter: Lindinger, Ingrid

(56) Entgegenhaltungen:
- EP-A- 0 237 952
- EP-A- 0 396 134
- EP-A- 0 722 935
- FR-A- 1 522 192
- US-A- 3 449 324

## Beschreibung

5H-Dibenzo-(b,f)-azepin, allgemein bekannt unter der Bezeichnung Iminostilben, ist ein Zwischenprodukt für pharmazeutisch wirksame Substanzen, wie etwa Carbamazepin.

Die Herstellung von Iminostilben erfolgt beispielsweise durch katalytische Dehydrierung von 10,11-Dihydro-5H-dibenzo-(b,f)-azepin, bekannt unter der Bezeichnung Iminodibenzyl. Insbesondere bei der katalytischen Dehydrierung in der flüssigen Phase oder in der Schmelze, wie beispielsweise in EP 0 237 952 oder EP 0 396 134 beschrieben, weist Iminostilben jedoch eine Vielzahl von Verunreinigungen, wie beispielsweise ein Dimerisierungsprodukt von Iminostilben, Methyliminostilben oder Acridine auf, die bei einer Weiterverarbeitung zu Carbamazepin nicht mehr zu entfernen sind.
Gemäß EP 0 237 952 wird nach erfolgter Umsetzung der Katalysator abfiltriert und Iminostilben ausgefällt. EP 0 396 134 weist darauf hin, dass Reproduktionsversuche des in EP 0 237 952 beschriebenen Verfahrens auf Grund der oben angeführten Nebenprodukte bzw. Verunreinigungen zu einem Produkt in schlechter Qualität führen. Jedoch weist auch das gemäß EP 0 396 134 hergestellte Iminostilben unumgesetztes 10,11-Dihydro-5H-dibenzo-(b,f)-azepin, Dimerisierungsprodukte und Acridine als Verunreinigungen auf. Die Aufarbeitung des Reaktionsgemisches erfolgt gemäß EP 0 396 134 ebenfalls durch Abfiltrieren des Katalysators und Ausfällen des Produktes durch Zugabe eines Lösungsmittels, in dem es sich schlecht löst, vorzugsweise Wasser. Das Endprodukt wird sodann nur noch gewaschen und getrocknet.
Wie Versuche und Erfahrungswerte zeigen, lassen sich diese Verunreinigungen durch bisher übliches, einfaches Umkristallisieren oder Aufschlämmen in einem aromatischen Kohlenwasserstoff, wie etwa Xylol oder Toluol, nicht aus dem Iminostilben entfernen.
Die Reinigung von Rohprodukten technischer Qualität oder von Produkten geringerer Qualität durch Umkristallisation oder Aufschlämmen in einem aromatischen Kohlenwasserstoff wird gemäß dem Stand der Technik unabhängig von dem verwendeten Herstellungsverfahren durchgeführt. Die dabei erzielte Reinheit ist jedoch keineswegs zufriedenstellend. Ein weiteres Problem, das bei der Umkristallisation unter Verwendung beispielsweise von aromatischen Kohlenwasserstoffen oder Acetaten auftritt, ist, dass das umkristallisierte Iminostilben durch die Bildung großer, dünner Plättchen unfiltrierbar wird.
Aus EP 0 722 935 ist bekannt, Iminostilben, hergestellt durch katalytische Dehydrierung, beispielsweise wie in EP 0 237 952 oder EP 0 396 134 vorgeschlagen, durch die Kombination zweier Reinigungsschritte aufzureinigen. Dabei wird rohes Iminostilben aus einem durch katalytische Dehydrierung von 10, 11-Dihydro-5H-Dibenzo-(b,f)-azepin erhaltenen Reaktionsgemisch im 1. Schritt unter reduziertem Druck verdampft und fest abgeschieden, das abgeschiedene Produkt im 2. Schritt in einem aromatischen Kohlenwasserstoff aufgeschlämmt und anschließend als reines 5H-Dibenzo-(b,f)-azepin isoliert.
Mit dieser Variante werden zwar Reinheiten von bis zu 99,6 GC-FI% erzielt, für die Weiterverarbeitung zu pharmazeutischen Substanzen, wie Carbamazepin, werden vom Markt jedoch noch höhere Reinheiten von mindestens 99, 85 bis zu 99,99 GC-FI.% gefordert.

Aufgabe der vorliegenden Erfindung war es demnach, ein geeignetes Verfahren zur Reinigung von Iminostilben, unabhängig von dessen Herstellungsweg, zu finden, mit dem eine ausreichend hohe Reinheit, bei gleichzeitigem Vermeiden von Ausbeuteverlusten, gewährleistet wird.

Unerwarteterweise konnte diese Aufgabe durch die Verwendung spezieller Lösungsmittelmischungen gelöst werden.

Gegenstand der vorliegenden Erfindung ist demnach ein Reinigungsverfahren zur Herstellung von hochreinem 5H-Dibenzo-(b,f)-azepin von mindestens 99,85 GC-FI%, das dadurch gekennzeichnet ist, dass rohes bzw. verunreinigtes 5H-Dibenzo-(b,f)-azepin in einem Lösungsmittelgemisch aus einem aromatischen Kohlenwasserstoff aus der Gruppe Toluol, Xylol, Benzol oder der Chlorbenzole mit
a) einem Alkohol aus der Gruppe Methanol, Ethanol, i-Propanol, n-Butanol, sek.Butanol, Cyclohexanol, Glycerin und Glycol oder
b) einem niedrigsiedenden Keton aus der Gruppe Aceton und Methylethylketon oder mit
c) einem aromatischen Kohlenwasserstoff aus der Gruppe Nitrotoluol und Toluidin,
oder in einem Lösungsmittelgemisch aus einem höhersiedenden Keton aus der Gruppe Methylisobutylketon, Diisopropylketon, Methylisopropylketon, Di-n-propylketon und Cyclohexanon mit
a) einem Alkohol aus der Gruppe Methanol, Ethanol, i-Propanol, n-Butanol, sek.Butanol, Glycerin, Glycol und Cyclohexanol oder
b) einem aromatischen Kohlenwasserstoff aus der Gruppe Nitrotoluol und Toluidin,
im Verhältnis 50:50 bis 98:2 umkristallisiert oder aufgeschlämmt wird und anschließend hochreines 5H-Dibenzo-(b,f)-azepin isoliert wird.

Bei dem erfindungsgemäßen Verfahren wird rohes bzw. verunreinigtes 5H-Dibenzo-(b,f)-azepin in hochreines 5H-Dibenzo-(b,f)-azepin überführt. Die Art und Weise der Herstellung des zu reinigenden Iminostilbens ist für das erfindungsgemäße Verfahren unerheblich. So kann beispielsweise Iminostilben, hergestellt durch katalytische Dehydrierung etwa gemäß EP 0 237 952 oder EP 0 396 134, oder aber ein durch ein beliebiges anderes Verfahren hergestelltes Iminostilben eingesetzt werden. Bei der Herstellung über katalytische Dehydrierung ist bereits eine Vielzahl von verschiedenen Varianten bekannt, bei denen verschiedenste Katalysatoren und/oder Wasserstoffakzeptoren verwendet werden und die beispielsweise in der Gasphase, in der Schmelze oder in der Flüssigphase durchgeführt werden. Andere Herstellungsvarianten sind beispielsweise die Herstellung aus Iminodibenzyl über 4 Stufen, die Acetylierung, Bromierung oder Chlorierung, Dehydrobromierung oder Dehydrochlorierung und Entacetylierung einschließen, die Herstellung aus Acridonmethanol in Gegenwart von Phosphorpentoxid u.s.w.. Das zu reinigende Iminostilben kann dabei einen Gehalt an Verunreinigungen von bis zu 15 GC- FI % aufweisen.

Zur erfindungsgemäßen Aufreinigung wird ein spezielles Lösungsmittelgemisch eingesetzt.

Als Lösungsmittelgemisch eignen sich dabei Gemische aus einem aromatischen Kohlenwasserstoff aus der Gruppe Toluol, Xylol, Benzol oder der Chlorbenzole mit
a) einem Alkohol aus der Gruppe Methanol, Ethanol, i-Propanol, n-Butanol, sek.Butanol, Cyclohexanol, Glycerin und Glycol oder
b) einem niedrigsiedenden Keton aus der Gruppe Aceton und Methylethylketon oder mit
c) einem aromatischen Kohlenwasserstoff aus der Gruppe Nitrotoluol und Toluidin,
sowie Gemische aus einem höhersiedenden Keton aus der Gruppe Methylisobutylketon, Diisopropylketon, Methylisopropylketon, Di-n-propylketon und Cyclohexanon mit
a) einem Alkohol aus der Gruppe Methanol, Ethanol, i-Propanol, n-Butanol, sek.Butanol, Glycerin, Glycol und Cyclohexanol oder
b) einem aromatischen Kohlenwasserstoff aus der Gruppe Nitrotoluol und Toluidin

Bevorzugte aromatische Kohlenwasserstoffe sind Toluol und Xylol, besonders bevorzugt ist Toluol.

Das Mischungsverhältnis von Kohlenwasserstoff bzw. Keton zu Alkohol, Keton oder Kohlenwasserstoff liegt zwischen 50:50 und 98:2 und ist abhängig von der Löslichkeit des Iminostilben im jeweils verwendeten Gemisch und von der gewählten Reinigungsvariante. Bei der Aufschlämmung ist ein Verhältnis von 60:40 bis 95:5 bevorzugt, während beim Umkristallisieren ein Verhältnis von 60:40 bis 90:10 bevorzugt ist.

Das zu reinigende Iminostilben kann erfindungsgemäß in einem der oben angeführten Lösungsmittelgemische umkristallisiert oder aufgeschlämmt werden.

Beim Aufschlämmen wird bei einer Temperatur von Raumtemperatur bis zur Rückflusstemperatur des eingesetzten Lösungsmittelgemisches gearbeitet. Bevorzugt wird bei einer Temperatur von 50°C bis zur Rückflusstemperatur, besonders bevorzugt bei der Rückflusstemperatur aufgeschlämmt.
Beim erfindungsgemäßen Aufschlämmen von IST gehen je nach Wahl des Lösungsmittelgemisches, Temperatur und Dauer bis zu etwa 99% des IST in Lösung. Je höher die Temperatur und Dauer des Erwärmens, desto größer ist der Anteil des gelösten IST. Bei Raumtemperatur wird IST nicht oder nur in geringen Mengen gelöst. Bevorzugt wird die Temperatur und die Dauer des Erwärmens so gewählt, dass 20 bis 98 % des IST, besonders bevorzugt 30 bis 95% des IST gelöst werden.
Das Reaktionsgemisch wird etwa 5 bis 360 min, bevorzugt 10 bis 240 min und besonders bevorzugt 20 bis 120 min bei der gewählten Temperatur gerührt. Längere Rührzeiten sind jedoch auch möglich.
Anschließend wird, falls erforderlich, bevorzugt auf 0 bis 40°C, besonders bevorzugt auf 10 bis 30°C abgekühlt, beispielsweise durch normale Kühlung über die Kesselwand oder durch Vakuumkristallisation, und das IST durch Abfiltrieren oder Zentrifugieren aus dem Lösungsmittelgemisch isoliert. Das isolierte IST wird sodann, bevorzugt mit dem selben Lösungsmittelgemisch wie für das Aufschlämmen verwendet wurde gewaschen, bevorzugt 1 bis 10mal, besonders bevorzugt 2 bis 5mal, und getrocknet. Die Mutterlauge kann dabei 1 bis 12mal, bevorzugt 3 bis 7mal, wieder eingesetzt bzw. recycliert werden.

Bei der erfindungsgemäßen Umkristallisation wird das zu reinigende IST solange bevorzugt auf der Rückflusstemperatur des gewählten Lösungsmittelgemisches gerührt, bis IST vollständig gelöst ist. Tiefere Temperaturen sind gewünschtenfalls auch möglich; es sind dabei aber niedrigere Durchsätze in Kauf zu nehmen. Anschließend wird wiederum auf 0 bis 40°C, bevorzugt auf 10 bis 30°C abgekühlt, gegebenenfalls noch einige Zeit bei dieser Temperatur gerührt und das IST durch Abfiltrieren und Zentrifugieren aus dem Lösungsmittelgemisch isoliert. Die weitere Aufarbeitung erfolgt analog dem Aufschlämmen.
Die Abkühlung kann auch hier durch normale Kühlung über die Kesselwand oder über Vakuumkristallisation erfolgen. Bei der Vakuumkristallisation wird bevorzugt auf ca. 80 bis 40°C abgekühlt, dann bis zu 70% des Lösungsmittelgemisches abdestilliert, filtriert und das auskristallisierte IST nachgewaschen. Bevorzugt wird zum Nachwaschen das Destillat anstelle von frischem Lösungsmittelgemisch verwendet, wodurch Lösungsmittel eingespart werden kann.

Bei allen Aufarbeitungsvarianten lassen sich die Ausbeuten an hochreinem IST durch Recyclierung der Mutterlaugen aus den Waschvorgängen steigern.

Durch das erfindungsgemäße Verfahren ist es, im Gegensatz zum Stand der Technik, problemlos möglich, das durch Umkristallisation gereinigte IST zu filtrieren. Der Vorteil des erfindungsgemäßen Aufschlämmens ist, dass im Vergleich zum Umkristallisieren sehr hohe Reinheiten und höhere Ausbeuten bei gleichzeitig hohem Durchsatz und geringerem Lösungsmittelverbrauch erzielt werden.
Weiters zeichnet sich das erfindungsgemäße Verfahren durch den einfachen Arbeitsablauf und durch die hohe erzielte Reinheit des IST von mindestens 99,85 GC-FI.% bis zu 99,99 GC-FI.% aus. Das erfindungsgemäß gereinigte IST entspricht somit den Marktanforderungen.

### Beispiel 1:

100g Rohiminostilben (95%ig)wurden in 700 ml Toluol/Isopropanol (85:15) bei Rückfluss aufgeschlämmt und eine Stunde bei dieser Temperatur gerührt. IST war bis zu 90% gelöst.
Anschließend wurde innerhalb 65 min auf 20°C abgekühlt und über einen Rosemundfilter (20µm Poromet, 20cm², 0,5-0,7 bar N₂) filtriert.
Die Filtrationszeit betrug 80 Sekunden, die Filterkuchenhöhe 10cm. Es wurden 590 ml Mutterlauge abgetrennt.
Anschließend wurde 4x mit je 50ml Toluol/lsopropanol (85:15) nachgewaschen. Dabei wurde die Waschlösung auf den Filterkuchen geleert, bei geschlossenem Ablasshahn 0,5 bar N₂ aufgedrückt und ohne aufzurühren filtriert. Die Filtrationszeiten betrugen zwischen 35 und 45 Sekunden.

Es wurde IST mit 99,94 GC-FI.% erhalten. (Ausbeute 85,4% bezgl. IST) Der Gehalt an Iminodibenzyl (unumgesetztes Ausgangsprodukt aus der Herstellung von IST) und an Acridin betrug je 0,01 GC-FI.%. Weiters waren 0,04 GC-FI.% an Methyl-IST vorhanden.

### Beispiel 2:

Analog Beispiel 1 wurden 125g Rohiminostilben (95%ig) in 500ml Toluol/Isopropanol (85:15) bei Rückfluss aufgeschlämmt und eine Stunde bei dieser Temperatur gerührt. IST war bis zu 70% gelöst.
Anschließend wurde innerhalb 65 min auf 20°C abgekühlt und über einen Rosemundfilter (20µm Poromet, 20cm², 0,5-0,7 bar N₂) filtriert.
Die Filtrationszeit betrug 75 Sekunden, die Filterkuchenhöhe 9cm. Es wurden 400 ml Mutterlauge abgetrennt.
Die Aufarbeitung erfolgte wiederum durch Nachwaschen mit 4x je 50ml Toluol/Isopropanol (85:15). Die Filtrationszeiten betrugen 9 bis 10 Sekunden.

Es wurde 106g (trocken, 88,5% bezgl. IST) IST mit 99,89 GC-FI.% erhalten. Iminodibenzyl war nicht nachweisbar. Der Gehalt an Acridin und Methyl-IST betrug je 0,04 GC-FI.%. Weiters waren 0,03 GC-FI.% an Methylacridin und 0,001 GC-FI.% an Diphenylamin nachweisbar.

### Beispiel 3:

Beispiel 2 wurde mit einem Methylisobutylketon/lsopropanol-Gemisch (70:30) wiederholt. (70% IST gelöst)
Es wurde IST mit 99,88 GC-FI.% erhalten. Der Gehalt an Iminodibenzyl und Acridin betrug 0,03 bzw. 0,04 GC-FI.%. Weiters waren 0,04 GC-FI.% an Methyl-IST nachweisbar. Methylacridin und Diphenylamin waren nicht nachweisbar.

### Beispiel 4:

1000g Rohiminostilben (95%ig) wurden in 2000ml Toluol/n-Butanol (95:5) bei Rückflusstemperatur (111°C) aufgeschlämmt und eine Stunde bei dieser Temperatur gerührt. (ca. 42% IST gelöst)
Die weitere Vorgangsweise entsprach Beispiel 1.
Es wurde IST mit 99,89 GC-FI.% erhalten. (Ausbeute 91,5% bezgl. IST) Der Gehalt an Iminodibenzyl betrug 0,0 28, der Gehalt an Acridin betrug 0,036 GC-FI.% und der Gehalt an Diphenylamin 0,002 GC-FI.%. Weiters waren 0,039 GC-FI.% an Methyl-IST vorhanden.

### Beispiel 5:

95 g Rohiminostilben (95%ig) wurden in 750ml Toluol/lsopropanol (85:15) bei Rückfluss gelöst. Das Gemisch mußte 2 Stunden auf Rückflusstemperatur gekocht werden, um IST vollständig zu lösen. Anschließend wurde innerhalb 60 min auf 20°C abgekühlt und über einen Rosemundfilter (20µm Poromet, 20cm², 0,5-0,7 bar N₂) filtriert. (Kristallisationsbeginn war bei 87,5°C)
Die Filtrationszeit betrug 200 Sekunden, die Filterkuchenhöhe 8cm. Es wurden 650 ml Mutterlauge abgetrennt.
Die Aufarbeitung erfolgte wiederum durch Nachwaschen mit 4x je 50ml Toluol/Isopropanol (85:15). Die Filtrationszeiten betrugen 9 bis 10 Sekunden.

Es wurde 75,4g (trocken, 83%) IST mit 99,96 GC-FI.% erhalten. Iminodibenzyl und Diphenylamin waren nicht nachweisbar. Der Gehalt an Acridin und Methylacridin lag unter 0,001 GC-FI.%. Weiters waren 0,036 GC-FI.% an Methyl-IST nachweisbar.

### Beispiel 6:

Beispiel 5 wurde mit einem Methylisobutylketon/Isopropanol-Gemisch (70:30) wiederholt. (IST vollständig gelöst)
Es wurde IST mit 99,94 GC-FI.% (Ausbeute 72,9% bezgl. IST) erhalten. Der Gehalt an Iminodibenzyl und Acridin betrug 0,01 bzw. 0,015 GC-FI.%. Weiters waren 0,037 GC-FI.% an Methyl-IST nachweisbar. Methylacridin und Diphenylamin waren nicht nachweisbar.

### Beispiel 7:

100 g Rohiminostilben (95%ig) wurden in 880ml Toluol/Isopropanol (80:20) bei Rückfluss gelöst und 1 Stunde auf Rückflusstemperatur (87°C) gekocht. Anschließend wurde der Heizungsschieber geschlossen und mit Vakuum auf 70°C abgekühlt. Dann wurden bis zu einer Innentemperatur von 45°C 520 ml Lösungsmittelgemisch abdestilliert. (Heizung: 70°C-40°C; Innentemperatur: 66°C-30°C; Druck: 450-55mbar; Dauer. 3,5 h) Ab einer Innentemperatur von 30°C wurde belüftet und mittels Thermostat auf 20°C abgekühlt, eine Stunde bei 20°C gerührt und dann über einen Rosemundfilter filtriert. (20µm Poromet, 20cm², 0,7-1,0 bar N₂). Die Filtrationszeit betrug 30 Sekunden. (Mutterlauge: 240ml; Filterkuchenhöhe 8cm) Anschließend wurde 4x mit je 130ml Destillat nachgewaschen. (Dauer 30-38 Sekunden) Dabei wurde die Waschlösung auf den Filterkuchen geleert, bei geschlossenem Ablasshahn 1 bar N₂ aufgedrückt und anschließend der Ablasshahn geöffnet. Das erhaltene IST wurde bei 50°C im Vakuumschrank getrocknet.
Es wurde IST mit 99,95 GC-FI.% (Ausbeute 89,6% bezgl. IST) erhalten. Der Gehalt an Iminodibenzyl und Acridin betrug je 0,004 GC-FI.%. Weiters waren 0,038 GC-FI.% an Methyl-IST nachweisbar. Methylacridin und Diphenylamin waren nicht nachweisbar.

## Patentansprüche

1. Reinigungsverfahren zur Herstellung von hochreinem 5H-Dibenzo-(b,f)-azepin mit einem Gehalt von mindestens 99,85 GC-FI%, **dadurch gekennzeichnet, dass** rohes bzw. verunreinigtes 5H-Dibenzo-(b,f)-azepin in einem Lösungsmittelgemisch aus einem aromatischen Kohlenwasserstoff aus der Gruppe Toluol, Xylol, Benzol oder der Chlorbenzole mit
a) einem Alkohol aus der Gruppe Methanol, Ethanol, i-Propanol, n-Butanol, sek.Butanol, Cyclohexanol, Glycerin und Glycol oder
b) einem niedrigsiedenden Keton aus der Gruppe Aceton und Methylethylketon oder mit
c) einem aromatischen Kohlenwasserstoff aus der Gruppe Nitrotoluol und Toluidin,
oder in einem Lösungsmittelgemisch aus einem höhersiedenden Keton aus der Gruppe Methylisobutylketon, Diisopropylketon, Methylisopropylketon, Di-n-propylketon und Cyclohexanon mit
a) einem Alkohol aus der Gruppe Methanol, Ethanol, i-Propanol, n-Butanol, sek.Butanol, Glycerin, Glycol und Cyclohexanol oder
b) einem aromatischen Kohlenwasserstoff aus der Gruppe Nitrotoluol und Toluidin im Verhältnis 50:50 bis 98:2 umkristallisiert oder aufgeschlämmt wird und anschließend hochreines 5H-Dibenzo-(b,f)-azepin isoliert wird.

2. Verfahren nach Anspruch 1, **dadurch gekennzeichnet, dass** das zu reinigende Iminostilben einen Gehalt an Verunreinigungen von bis zu 15 GC- Fl % aufweist.

3. Verfahren nach Anspruch 1, **dadurch gekennzeichnet, dass** bei der Aufschlämmung ein Lösungsmittelgemisch im Verhältnis von 60:40 bis 95:5 und beim Umkristallisieren ein Lösungsmittelgemisch im Verhältnis von 60:40 bis 90:10 eingesetzt wird.

4. Verfahren nach Anspruch 1, **dadurch gekennzeichnet, dass** das zu reinigende Iminostilben beim Aufschlämmen bei einer Temperatur von Raumtemperatur bis zur Rückflusstemperatur des eingesetzten Lösungsmittelgemisches solange auf dieser Temperatur gehalten wird bis 20 bis etwa 99 % des Iminostilbens gelöst sind.

5. Verfahren nach Anspruch 1, **dadurch gekennzeichnet, dass** das zu reinigende Iminostilben beim Umkristallisieren solange auf Rückflusstemperatur des eingesetzten Lösungsmittelgemisches wird, bis das Iminostilbens vollständig gelöst ist.

6. Verfahren nach Anspruch 1, **dadurch gekennzeichnet, dass** zur Isolierung des hochreinen Iminostilbens das Gemisch nach dem Aufschlämmen oder Umkristallisieren gegebenenfalls auf 0 bis 40°C abgekühlt, das gereinigt Iminostilben filtriert und gewaschen wird.

7. Verfahren nach Anspruch 8, **dadurch gekennzeichnet, dass** zur Isolierung des hochreinen Iminostilbens nach erfolgter Umkristallisation das Abkühlen auf 80 bis 40°C mittels Vakuumkristallisation erfolgt, anschließend bis zu 70% des Lösungsmittelgemisches abdestilliert wird und gereinigtes Iminostilben mit dem Destillat nachgewaschen wird.

## Claims

1. Purification process for the preparation of highly pure 5H-dibenzo[b,f]azepine having a content of at least 99.85 GC area%, **characterized in that** it comprises recrystallizing or suspending crude or impure 5H-dibenzo[b,f]azepine in a solvent mixture of an aromatic hydrocarbon from the group toluene, xylene, benzene or chlorobenzenes with
a) an alcohol from the group methanol, ethanol, i-propanol, n-butanol, sec-butanol, cyclohexanol, glycerol and glycol or
b) a low-boiling ketone from the group acetone and methyl ethyl ketone or with
c) an aromatic hydrocarbon from the group nitrotoluene and toluidine,
or in a solvent mixture of a relatively high-boiling ketone from the group methyl isobutyl ketone, diisopropyl ketone, methyl isopropyl ketone, di-n-propyl ketone and cyclohexanone with
a) an alcohol from the group methanol, ethanol, i-propanol, n-butanol, sec-butanol, glycerol, glycol and cyclohexanol or
b) an aromatic hydrocarbon from the group nitrotoluene and toluidine,
in the ratio 50:50 to 98:2 and then isolating highly pure 5H-dibenzo[b,f]azepine.

2. Process according to Claim 1, **characterized in that** the iminostilbene to be purified has a content of impurities of up to 15 GC area%.

3. Process according to Claim 1, **characterized in that** a solvent mixture in the ratio from 60:40 to 95:5 is employed in the case of suspension and a solvent mixture in the ratio from 60:40 to 90:10 is employed in the case of recrystallization.

4. Process according to Claim 1, **characterized in that** the iminostilbene to be purified in the suspension at a temperature from room temperature up to the reflux temperature of the solvent mixture employed is kept at this temperature until up to 20 to approximately 99% of the iminostilbene is dissolved.

5. Process according to Claim 1, **characterized in that** the iminostilbene to be purified in the recrystallization is [lacuna] at the reflux temperature of the solvent mixture employed until the iminostilbene is completely dissolved.

6. Process according to Claim 1, **characterized in that**, for the isolation of the highly pure iminostilbene, the mixture after the suspension or recrystallization is optionally cooled to 0 to 40°C, and the purified iminostilbene is filtered and washed.

7. Process according to Claim 8, **characterized in that**, for the isolation of the highly pure iminostilbene, after recrystallization has taken place cooling to 80 to 40°C by means of vacuum crystallization takes place, and up to 70% of the solvent mixture is then distilled off and purified iminostilbene is subsequently washed with the distillate.

## Revendications

1. Procédé de purification pour la fabrication de 5H-dibenzo-(b,f)-azépine très pure d'une teneur d'au moins 99,85 CG-IF %, **caractérisé en ce que** la 5H-dibenzo-(b,f)-azépine brute et, impure est recristallisée ou mise en suspension dans un mélange de solvants constitué d'un hydrocarbure aromatique appartenant au groupe : toluène, xylène, benzène ou chlorobenzènes avec
a) un alcool appartenant au groupe : méthanol, éthanol, i-propanol, n-butanol, sec-butanol, cyclohexanol, glycérine et glycol ou
b) une cétone de bas point d'ébullition appartenant au groupe : acétone et méthyléthylcétone ou avec
c) un hydrocarbure aromatique appartenant au groupe : nitrotoluène et toluidine.
ou dans un mélange de solvants constitué d'une cétone de point d'ébullition plus élevé appartenant au groupe : méthylisobutylcétone, diisopropylcétone, méthylisopropylcétone, di-n-propylcétone et cyclohexanone avec
a) un alcool appartenant au groupe : méthanol, éthanol, i-propanol, n-butanol, sec-butanol, glycérine,glycol et glycohexanol ou
b) un hydrocarbure aromatique appartenant au groupe : nitrotoluène et toluidine dans un rapport de 50/50 à 98/2 et qu'on isole ensuite la 5H-dibenzo-(b,f)-azépine très pure.

2. Procédé selon la revendication 1, **caractérisé en ce que** l'iminostilbène à purifier présente une teneur en impuretés pouvant atteindre 15 CG-IF %.

3. Procédé selon la revendication 1, **caractérisé en ce que** on utilise pour la mise en suspension un mélange de solvants dans un rapport de 60/40 à 95/5 et pour la recristallisation un mélange de solvants dans un rapport de 60/40 à 90/10.

4. Procédé selon la revendication 1, **caractérisé en ce que** l'on maintient l'iminostilbène à purifier au cours de la mise en suspension à une température allant de la température ambiante à la température de reflux du mélange de solvants utilisé, à cette température jusqu'à ce que 20 à environ 99 % de l'iminostilbène soient dissous.

5. Procédé selon la revendication 1, **caractérisé en ce que** l'iminostilbène à purifier est maintenu au cours de la recristallisation à la température de reflux du mélange de solvants utilisé, jusqu'à ce que l'iminostilbène soit entièrement dissous.

6. Procédé selon la revendication 1, **caractérisé en ce que** l'on refroidit le mélange, pour l'isolement de l'iminostilbène très pur, après la mise en suspension ou la recristallisation, le cas échéant à 0 - 40 °C, on filtre l'iminostilbène purifié et on le lave.

7. Procédé selon la revendication 8, **caractérisé en ce que**, pour l'isolement de l'iminostilbène très pur une fois la recristallisation terminée, on refroidit à 80 - 40°C par cristallisation sous vide, on distille ensuite le mélange de solvants jusqu'à 70% et on lave l'iminostilbène purifié avec le distillat.
